Europäisches Patentamt

European Patent Office

Office européen des brevets

(11) Publication number: **0 209 900**
A2

(12) **EUROPEAN PATENT APPLICATION**

(21) Application number: 86110131.9

(22) Date of filing: 23.07.86

(51) Int. Cl.⁴: **G 01 N 11/00**
**G 01 N 3/22, G 01 N 3/26**
**G 01 N 25/04**

(30) Priority: 24.07.85 IT 6767685

(43) Date of publication of application:
28.01.87 Bulletin 87/5

(84) Designated Contracting States:
DE FR GB NL SE

(71) Applicant: CSELT Centro Studi e Laboratori
Telecomunicazioni S.p.A.
Via Guglielmo Reiss Romoli, 274
I-10148 Turin(IT)

(72) Inventor: Cocito, Giuseppe
Via M. D'Azeglio, 15 - S.
Giusto Can.se Torino(IT)

(74) Representative: Riederer Freiherr von Paar zu Schönau,
Anton et al,
Freyung 615 Postfach 2664
D-8300 Landshut(DE)

(54) Method and apparatus for measuring the softening point of a vitreous specimen.

(57) The method and apparatus for measuring the softening point of a vitreous specimen detect viscosity variations of the specimen under temperature variations through measurement of mechanical deformations due to the application of a torque. The softening point is found in the temperature interval where the viscosity drops.

EP 0 209 900 A2

**VAN DER WERTH, LEDERER & RIEDERER**
Patentanwälte

DR. A. VAN DER WERTH
(1934 – 1974)

DR. FRANZ l **0209900**
Dipl. Chem.

ANTON FREIHERR
RIEDERER v. PAAR
Dipl.-Ing.                    Landshut

D-8300 Landshut
Postfach 2664, Freyung 615

☎ (08 71) 2 21 70
Telefax (CCITT 2) manuell
Telex 58441 glala d

Frhr. Riederer v. Paar, Postfach 2664, D-8300 Landshut

CSELT

Centro Studi e Laboratori

Telecomunicazioni S.p.A.

Turin, Italy

## Method And Apparatus For Measuring The Softening Point Of A Vitreous Specimen

## Description

The present invention concerns measurements for the characterization of vitreous materials and more particularly a method and an apparatus for measuring the softening point of a specimen made of the above materials.

As known, vitreous-material characterization requires the knowledge of various parameters, namely the softening point, or the temperature at which the material attains sufficiently low viscosity values. Under these conditions the material can no longer be considered as solid. Actually, the change of state does not occur at a determined temperature, but a considerable viscosity variation takes place within a temperature interval.

A viscosity curve as a function of temperature can be plotted which basically consists of a first high-viscosity segment, a second segment in which the transition occurs, and a third low-viscosity segment.

In case of an optical fibre, the softening point is to be determined in order to evaluate the possibility of carrying out some particular operations, such as the making of "fusion joints". Said term refers to optical fibre joints produced by raising the temperature of fibre ends to the softening point and bringing them in contact so that they are soldered. If the fibres to be joined are different, the problem of their compatibility arises. The difference between their softening points is to be minimum, since it usually is indicative of the different vitreous compositions and hence of the different parameters affecting the joint quality. If the fibre compatibility is low, residual stresses in the fusion zone arise and make the joint very fragile.

An apparatus for measuring a glass-fibre softening point is described in the Tutorial Symposium Proceedings, New York, 8-9 June 1970, pages 355-357. A determined-length cylinder (or rod) hangs inside a furnace and the lengthening due to its weight is measured by suitable optical detectors.

The temperature is increased according to a particular law and the softening point is determined by the temperature at which the lengthening rate of the fibre attains a pre-determined value.

However, this apparatus provides low accuracy and measurement repeatability. In fact, when the specimen under test attains the softening point, it begins to lengthen and its cross section decreases as a function of weight, of surface strains and of the rate of increase in furnace temperature.

As a consequence, the initial conditions change and the results are always different and less and less reliable for higher specimen temperature and lengthening values.

These disadvantages are overcome by the method and apparatus for measuring the softening point of a vitreous specimen, provided by the present invention, which allow accurate detection of the viscosity curve in function of temperature, without causing longitudinal deformations of the specimen under test, and provide a measurement method which can be used with both decreasing and increasing temperatures.

The present invention provides a method of measuring the softening point of a vitreous specimen in which the viscosity changes of the specimen under temperature variations are detected from the measurement of mechanical deformations, characterized in that the specimen above undergoes torsion and the torque variations with varying temperature are recorded, the softening point being found in the temperature interval in which viscosity drops.

The invention also provides the apparatus for implementing the method.

These and other features of the present invention will be apparent from the following description of a preferred embodiment thereof, given by way of example and not in a limiting sense, with reference to the annexed drawing showing the apparatus under schematic form.

The apparatus uses a measurement method which allows viscosity variations of the specimen subjected to temperature variation to be detected from the measurement of torsion deformations.

The apparatus consists of a torque meter for measuring the torque exerted by the specimen under test, i.e. the optical fibre FO, clamped by a mandrel M1 integral with the meter. A second mandrel M2 secures the other fibre end in order to apply the desired torque through a lockable knob connected to it and to a protractor G. Fibre FO traverses a small-size furnace F, which operates up to a temperature of about 1800°C and is equipped with a thermometric probe for operating temperature readings.

To effect the measurement, the unclad fibre is clamped between the two mandrels and is twisted to a certain angle in function of its diameter and structure. Then the furnace is switched on and temperature and torque are plotted versus time. The recording can be repeated passing from high to low temperature values. The softening point of vitreous specimens can thus be located exactly in the interval of viscosity drop.

By a suitable choice of the torsion angle, the deformations the fibres undergo can be reduced at will, hence the results are reliable in a wide temperature range.

It is clear that the above description has been given only by way of a non limiting example. Variations and modifications are possible without going beyond the scope of the invention.

For example, the structure supporting the different parts of the apparatus can present any number of different configurations, and the mandrels can be adapted to suit the form of the vitreous specimens involved.

Thus the apparatus is suited to a procedure which can be completely automated and possibly controlled by a programmed microprocessor, so as to automatically extract curve families fully representing the vitreous material softening phenomenon.

## 1 Claims

1.  A method of measuring the softening point of a vitreous specimen, in which the viscosity changes of the specimen under temperature variations are detected from the measurement of its mechanical deformations, characterized in that the specimen above undergoes torsion and the torque variations with varying temperatures are recorded, the softening point being found in the temperature interval in which viscosity drops.

2.  Apparatus to implement the method of claim 1, which detects the viscosity changes of the specimen under temperature variations from the measurement of its mechanical deformations characterized in that it consists of:

    - a torque meter (RT);

    - a first mandrel (M1) integral with the torque meter, to clamp an end of the specimen under test;

    - a furnace (F) traversed by the specimen under test, equipped with a thermometric probe;

    - a second mandrel (M2), to clamp the other end of the specimen under test;

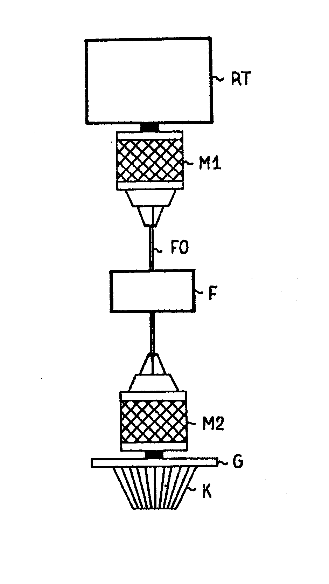

    - a protractor (G), integral with the second mandrel which can be locked in the desired position.

1\1